# EUROPEAN PATENT APPLICATION

(11) **EP 1 103 280 A1**
(43) Date of publication of application: **30.05.2001**
(21) Application number: 00308994.3
(22) Date of filing: 12.10.2000
(51) Int. Cl.: A61M 25/00

(54) **Cuffed medico-surgical tubes**

(30) Priority: 26.11.1999 GB 9927814
(71) Applicant: Smiths Industries Public Limited Company, London, NW11 8DS (GB)
(72) Inventor: Veasey, Neil, Folkestone, Kent CT19 4LU (GB)
(74) Representative: Flint, Jonathan McNeill

(57) **Abstract**

A cuffed tracheal tube has a helically-reinforced shaft 2, 102, 202, 302 and a separate, unreinforced tip 9, 109, 209, 309 joined at its patient end by an abutting or overlapping join. The join between the tip and shaft is improved because the patient end collar 23, 123, 223, 321 of the cuff 20, 120, 220, 320 overlaps the join or abuts an overlapping machine end of the tip 9, 109, 209, 309. The cuff 220 may be moulded integrally with the tip 209.

## Description

This invention relates to cuffed medico-surgical tubes of the kind including a tube shaft, a separate patient end tip joined with the tube shaft and an inflatable cuff surrounding the tube towards the patient end.

Cuffed tracheal tubes are used to ventilate and administer anaesthesia to patients, the cuff being inflated to seal with the inside of the trachea. In some cases, it is preferable for the patient end tip of the tube to be of a different material, such as a softer material, from the remainder of the tube shaft. Where the main part of the tube shaft is reinforced with a helical reinforcement element, it can be advantageous to have an unreinforced patient end tip, in order to minimize the risk that the reinforcing element will be exposed at the tip and cause trauma. Although there is no problem in joining a tip of a separate material to the tube shaft, it is important to ensure that the risk of the tip becoming detached from the tube during use is minimized. It is also important to ensure that the external surface of the tube is as smooth as possible.

It is an object of the present invention to provide an alternative tube.

According to the present invention there is provided a cuffed medico-surgical tube of the above-specified kind, characterised in that the patient end of the cuff adjoins with the machine end of the patient end tip.

The machine end of the patient end tip may overlap the outside of the patient end of the tube shaft, the patient end of the cuff abutting the machine end of the patient end tip on the outside of the tube shaft. Alternatively, the machine end of the patient end tip may overlap the outside of the patient end of the tube shaft, the patient end of the cuff overlapping the outside of the machine end of the patient end tip. Alternatively, the cuff and patient end tip may be a single integral component. The cuff and patient end tip may be formed by injection moulding. In a further alternative arrangement the machine end of the patient end tip may abut the patient end of the tube shaft, the patient end of the cuff overlapping the join between the shaft and the patient end tip. The tube shaft is preferably reinforced, such as by a helical reinforcing element, and the patient end tip is preferably unreinforced.

Four cuffed tracheal tubes according to the present invention, will now be described, by way of example, with reference to the accompanying drawings, in which:
- Figure 1: is a side elevation view of a first form of tracheal tube;
- Figure 2: is a sectional side elevation view of the patient end of the tube of Figure 1 to a larger scale;
- Figure 3: is a sectional side elevation view of the patient end of an alternative tube;
- Figure 4: is a sectional side elevation view of the patient end of a third form of tube; and
- Figure 5: is a sectional side elevation view of the patient end of a fourth form of tube.

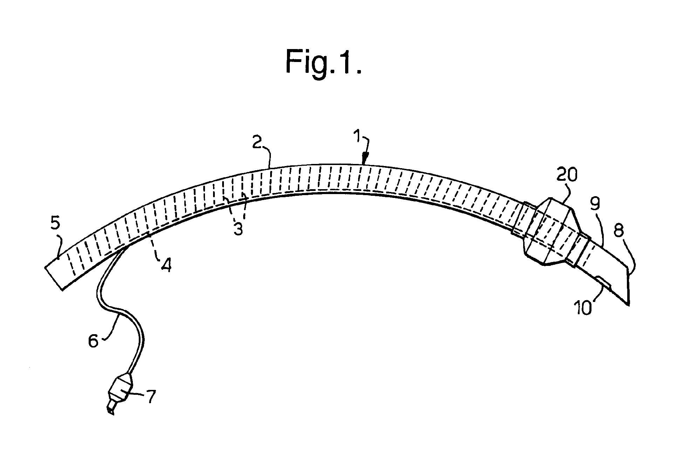

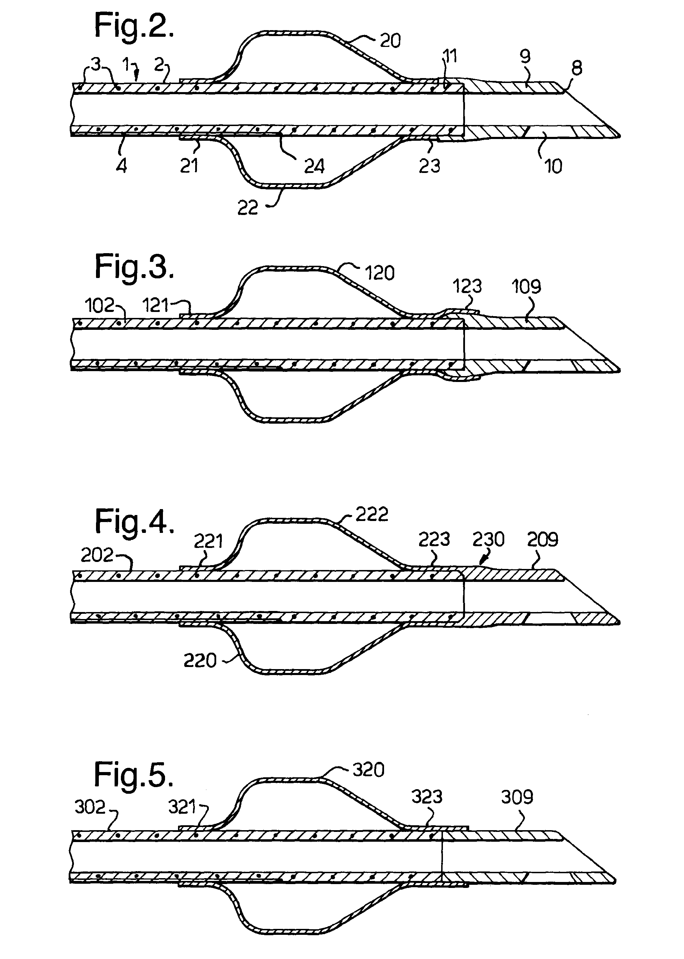

With reference first to Figures 1 and 2, the tracheal or endotracheal tube 1 has a shaft 2 reinforced in a conventional manner, such as with a helical reinforcing element 3. The shaft 2 has a small-bore inflation lumen 4 extending along its length within the thickness of the wall of the shaft. Towards the machine end 5 of the tube 1 this joins with one end of an inflation line 6, which is joined at its other end with an inflation indicator and connector 7.

At its patient end 8, the tube 1 is bevelled, the patient end tip 9 of the tube being a separate component joined to the patient end of the shaft 2. The patient end tip 9 is unreinforced so as to provide a soft, atraumatic tip to the patient end of the tube. The patient end tip 9 is preferably made by moulding and optionally is formed with a side opening or Murphy eye 10. Along most of its length, the internal and external diameter of the patient end tip 9 is the same as that of the main shaft 2, except at its machine end where it is formed with an internal step 11 having an internal diameter equal to the external diameter of the shaft, the patient end of the shaft being inserted within the stepped machine end of the tip so that the machine end of the tip overlaps the outside of the patient end of the shaft. The external surface of the patient end tip 9 is smoothed in the region of the step 11, to make it atraumatic. The patient end tip 9 is securely bonded onto the patient end of the main shaft 2, such as by means of a solvent or by using heat.

The tube is completed by an inflatable cuff 20 of conventional form, comprising a machine end collar 21, a central inflatable portion 22 and a patient end collar 23. The two collars 21 and 23 are securely bonded to the outside of the shaft 2 using a solvent or the like so that the inflatable portion 22 is located over an opening 24 from the inflation lumen 4 on the outside of the tube. The patient end collar 23 is positioned so that it abuts the machine end of the patient end tip 9 where this extends along the outside of the shaft 2. Abutting the cuff 20 with the patient end tip 9 makes the cuff adjoin the tip and thereby reduces the height of the transition or step between the machine end of the patient end tip and the shaft, it also reduces the number of interruptions or transitions on the external surface of the tube. A further advantage is that flow of solvent, adhesive or melted material between the cuff 20 and the patient end tip 9 joins the patient collar of the cuff with the machine end of the patient end tip and thereby improves the bond of the patient end tip on the shaft 2. This arrangement, therefore, enables a highly secure join to be formed between the patient end tip 9 and the main shaft 2 whilst retaining a substantially smooth external surface.

Figure 3 shows a modification of the arrangement in Figure 2 where similar components to those in Figure 2 have been given the same number plus 100. Instead of butting the end of the cuff against the end of the patient end tip, as shown in Figure 2, the patient end collar 123 of the cuff 120 overlaps the machine end of patient end tip 109. This arrangement produces a single transition on the exterior at the patient end of the cuff 120. The overlap of the cuff 120 on the patient end tip 109 produces additional security of retention of the tip.

With reference now to Figure 4, there is shown another arrangement in which the cuff adjoins and is contiguous with the patient end tip. Similar components to those in Figure 2 have been given the same number plus 200. In this arrangement, the cuff 220 and patient end tip 209 are a single, one-piece, integral component 230 formed by injection moulding. The component 230 has a patient end portion or tip 209 and a machine end providing the cuff 220, which is joined with the external surface of the shaft 202 to the rear of the join of the tip with the shaft. The patient end tip 209 has an internal and external diameter along its entire length matched with those of the main shaft and makes a butt join with the patient end of the shaft 202. The cuff portion 220 has a machine end collar 221, a central inflatable portion 222 and a patient end collar 223, which is a continuation of the patient end tip 209. Because the cuff 220 and patient end tip 209 are integral with one another, the cuff helps improve the security of the join with the shaft 202. This arrangement has a completely smooth external surface from the patient end to the machine end of the cuff.

With reference now to Figure 5 there is shown a fourth form of the tube in which the patient end tip 309 makes a simple butt join with the shaft 302 and in which the patient end collar 323 of the cuff 320 overlaps the butt join. The collar 323 is thereby attached to both the patient end of the shaft 302 and the machine end of the patient end tip 309. Instead of a simple butt join with flat ends, other forms of join could be used such as involving inserted male and female extensions, dovetails or the like.

The invention is not confined to tracheal tubes but could be applied to other cuffed tubes where it is desirable to have a patient end tip different from the main shaft.

## Claims

1. A cuffed medico-surgical tube including a tube shaft (2, 102, 202, 302), a separate patient end tip (9, 109, 209, 309) joined with the tube shaft and an inflatable cuff (20, 120, 220, 320) surrounding the tube towards the patient end, characterised in that the patient end (23, 123, 223, 323) of the cuff (20, 120, 220, 320) adjoins with the machine end of the patient end tip (9, 109, 209, 309).

2. A tube according to Claim 1, characterised in that the machine end of the patient end tip (9) overlaps the outside of the patient end of the tube shaft (2), and that the patient end (23) of the cuff (20) abuts the machine end of the patient end tip (9) on the outside of the tube shaft (2).

3. A tube according to Claim 1, characterised in that the machine end of the patient end tip (109) overlaps the outside of the patient end of the tube shaft (102), and that the patient end (123) of the cuff (120) overlaps the outside of the machine end of the patient end tip (109).

4. A tube according to Claim 1, characterised in that the cuff (222) and patient end tip (209) are a single integral component (230).

5. A tube according to Claim 4, characterised in that the cuff (222) and patient end tip (209) are formed by injection moulding.

6. A tube according to Claim 1, characterised in that the machine end of the patient end tip (309) abuts the patient end of the tube shaft (302), and that the patient end (323) of the cuff (320) overlaps the join between the shaft (302) and the patient end tip (309).

7. A tube according to any one of the preceding claims, characterised in that the tube shaft (2, 102, 202, 302) is reinforced and the patient end tip (9, 109, 209, 309) is unreinforced.

8. A tube according to any one of the preceding claims, characterised in that the tube shaft (2, 102, 202, 302) is reinforced by a helical reinforcing element (3).
